# FASCICULE DE BREVET EUROPEEN

(11) **EP 1 984 071 B1**
(45) Date de publication et mention de la délivrance du brevet: **09.12.2009**
(21) Numéro de dépôt: 07730823.7
(22) Date de dépôt: 15.01.2007
(51) Int. Cl.: A61N 1/05

(54) **CATHETER DE NEUROSTIMULATION**
NERVENSTIMULATIONSKATHETER
NEUROSTIMULATION CATHETER

(30) Priorité: 02.02.2006 FR 0600948
(43) Date de publication de la demande: 29.10.2008
(73) Titulaire: VYGON, F-95440 Ecouen (FR)
(72) Inventeur: CARREZ, Jean-Luc, F-95440 Ecouen (FR); DALLE, Valérie, F-60270 Gouvieux (FR); GUYOMARC'H, Pierrick, F-95120 Ermont (FR); DULONG, Claire, F-60126 Rivecourt (FR); GADENNE, Cécile, F-92500 Rueil Malmaison (FR)
(74) Mandataire: Le Forestier, Eric
(86) Numéro de dépôt international: PCT/FR2007/000064
(87) Numéro de publication internationale: WO 2007/088256

(56) Documents cités:
- EP-A- 1 605 729
- US-A- 5 275 597
- US-A- 5 372 603
- US-A- 5 599 295
- US-A- 6 134 476
- US-A1- 2004 039 434
- US-A1- 2005 150 762

## Description

L'invention concerne un cathéter de neurostimulation constitué par un tube souple en matériau non conducteur de l'électricité dont l'extrémité distale est arrondie et qui comprend un conducteur électrique qui s'étend dans la longueur du tube pour conduire un courant électrique de stimulation d'une extrémité à l'autre du tube.

La neurostimulation permet au praticien de s'assurer que le cathéter a atteint l'objectif désiré par la réaction du bloc plexique à une excitation électrique provenant de l'extrémité distale du cathéter.

Pour rendre neurostimulable un tube cathéter non conducteur de l'électricité, il est connu d'adjoindre au tube un conducteur électrique qui s'étend sensiblement dans la longueur du cathéter entre un contact électrique en liaison avec le conducteur à l'extrémité distale du tube pour le contact du conducteur avec les nerfs ou les tissus de corps et un raccord électrique en liaison avec le conducteur à l'extrémité proximale du tube pour l'alimentation électrique du conducteur.

Le conducteur peut être constitué par un mandrin métallique introduit dans la lumière du tube et qui doit être retiré après mise en place du tube pour dégager la lumière avant la mise en service du cathéter, comme décrit par exemple dans les publications GB 88 03 153, DE 1 807 487 et CA 2 260 080.

Le conducteur peut aussi être incorporé à demeure au tube pour permettre la stimulation à tout moment, comme décrit par exemple dans les publications DE 101 00976, US 5 081 990.

L'incorporation permanente d'un conducteur métallique pose un problème d'encombrement si le conducteur est placé à l'intérieur du tube et complique la fabrication du tube si le conducteur est incorporé dans la paroi du cathéter.

L'invention vise à éviter ces inconvénients.

On y parvient selon l'invention en constituant le conducteur par au moins une couche d'un matériau conducteur de l'électricité appliquée sur la face externe de la paroi latérale du tube et isolée par un revêtement non conducteur appliqué sur la couche, ladite couche conductrice formant à l'extrémité distale du tube un anneau distal qui épouse la forme de l'arrondi de l'extrémité distale du tube et qui n'est pas recouvert par le revêtement isolant de la couche de façon à constituer un contact de neurostimulation, et ladite couche conductrice formant à l'extrémité proximale du tube un anneau proximal qui n'est pas recouvert par le revêtement isolant pour réaliser une connexion électrique.

De cette façon, la lumière du tube reste totalement libre et la fabrication du tube reste simple.

On utilise par exemple une encre utilisée pour le traçage argenté de pistes électriques au stylo et un revêtement acrylique résistant également appliqué au stylo ; on peut utiliser en variante un adhésif époxy contenant des particules d'argent, ou une encre conductrice sans liant qui en séchant ne laisse que le métal. Ces exemples ne sont pas exclusifs d'autres choix.

L'application se fait au stylo, ou de préférence par sérigraphie ou tampographie.

On a déjà proposé d'appliquer une encre conductrice sur la face externe de la paroi d'un cathéter (WO/2005/060870) mais essentiellement dans le but de pouvoir mettre en oeuvre un procédé électrostatique pour l'application précise d'un médicament sur la paroi du cathéter.

On a également proposé (brevet US 5 372 603) de munir un cathéter à ballon destiné à une application cardiovasculaire, de deux couches conductrices séparées pour transmettre de la puissance électrique à un élément associé au ballon.

Dans des modes de réalisation préférés, le cathéter de l'invention présente encore une ou plusieurs des caractéristiques suivantes :
•la couche conductrice constitue une ligne de largeur et d'épaisseur calibrées
• un tube conducteur fendu est enfilé sur l'extrémité proximale du cathéter pour réaliser une connexion électrique avec l'anneau proximal ;
• la paroi du tube conducteur est découpée longitudinalement pour former au moins une languette élastique destinée à assurer un contact électrique entre le tube et un fil électrique maintenu au contact de la languette ;
• le cathéter est muni d'une embase proximale ;
• un raccord est rapporté sur l'extrémité proximale du tube pour maintenir le fil conducteur ;
• ladite couche est constituée par une encre contenant de l'argent ;
• ladite couche est constituée par un mélange de solvant et de poudre d'argent
• ladite couche a été appliquée par sérigraphie, par tampographie ou par jet d'encre.

On décrira ci-après des exemples de réalisation d'un cathéter et d'une embase selon l'invention, en référence aux figures jointes sur lesquelles :
- la figure 1 est un schéma de principe du cathéter ;
- la figure 2 est une perspective de l'extrémité proximale du cathéter selon un mode de réalisation ;
- la figure 3 est une coupe longitudinale d'une réalisation d'une embase pour le cathéter ;
- la figure 4 est une perspective du corps tubulaire de l'embase ;
- la figure 5 est une vue de détail du tube conducteur à rapporter sur l'extrémité proximale de la partie tubulaire du cathéter selon une réalisation de l'invention ;
- la figure 6 est une vue éclatée ;
- la figure 7 est une perspective du cathéter avec son embase, prêt à être utilisé ;
- la figure 8 est une coupe axiale d'une variante de réalisation de l'embase du tube cathéter, avant la mise en place du cathéter dans l'embase et le raccordement électrique ;
- la figure 9 est une perspective de l'embase de la figure 8 ;
- la figure 10 montre la même coupe que celle de la figure 8 après la mise en place du cathéter dans l'embase, serrage de l'écrou et raccordement électrique ;
- la figure 11 est une perspective de l'embase de la figure 10, et
- la figure 12 est une coupe transversale de l'embase selon la variante de réalisation au niveau du raccordement électrique.

Le cathéter représenté sur les figures est constitué par un tube souple (1) en matériau non conducteur de l'électricité sur la face extérieure duquel a été appliquée selon une ligne (2) une couche (A) d'un matériau conducteur de l'électricité qui s'étend de l'extrémité proximale (1a) à l'extrémité distale (1b) du tube (1) et qui est revêtu localement d'une couche d'un matériau isolant (B).

A ses extrémités, la couche conductrice forme deux anneaux (3,4) de quelques millimètres de longueur et le revêtement isolant (B) a été arrêté avant ces anneaux.

L'un des anneaux servira à l'alimentation électrique du revêtement conducteur et l'autre anneau servira à transmettre l'excitation électrique aux nerfs (bloc plexique) à l'endroit où on veut stimuler ou repérer les nerfs.

Entre les deux anneaux, il ne faut pas que le corps humain soit en contact avec le conducteur d'où la présence d'un revêtement isolant (à base de plastique et de solvant par exemple) sur la ligne conductrice, de manière à ce qu'elle soit complètement recouverte. De cette manière, les deux anneaux conducteurs sont reliés par un conducteur isolé.

L'anneau distal conducteur, en tant que contact électrique, est une bonne solution pour neurostimuler car il est peu encombrant (épaisseur 15 à 20 µm), et n'engendre donc pas de grosse surépaisseur qui pourrait gêner le passage du cathéter dans une aiguille ; il permet d'utiliser un cathéter avec un trou distal non bouché et non rétréci, ce qui est mieux pour le débit. Cela évite aussi d'avoir à pousser fort sur la seringue d'injection.

Si le cathéter est émoussé (bout arrondi) côté distal, l'anneau peut épouser la forme de l'arrondi (atraumatique).

L'invention concerne également des réalisations d'une embase à monter sur l'extrémité proximale d'un cathéter de neurostimulation, en particulier d'un cathéter selon l'invention, équipée de moyens pour maintenir le cathéter dans l'embase et de moyens pour assurer une connexion électrique entre un conducteur d'alimentation électrique et le revêtement conducteur du cathéter, et apte au raccordement du cathéter avec un dispositif d'alimentation d'une solution, par exemple d'une seringue d'injection.

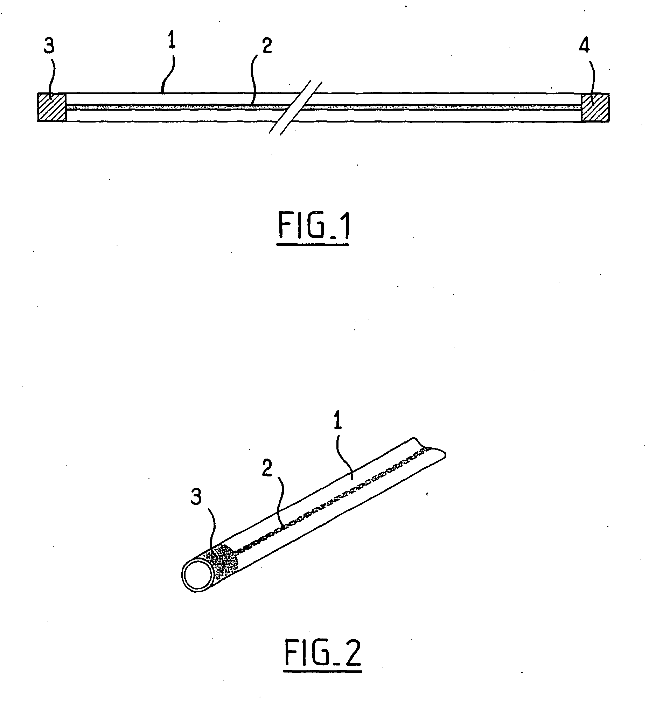

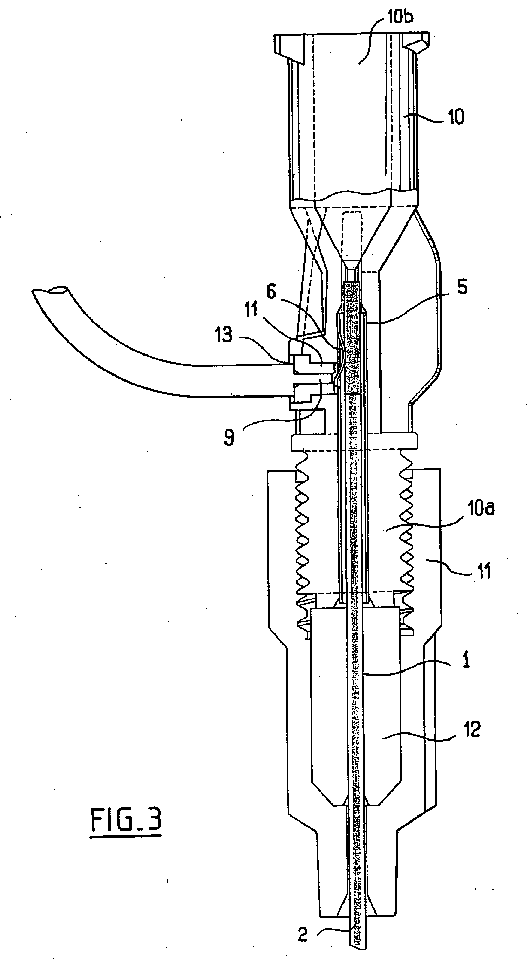

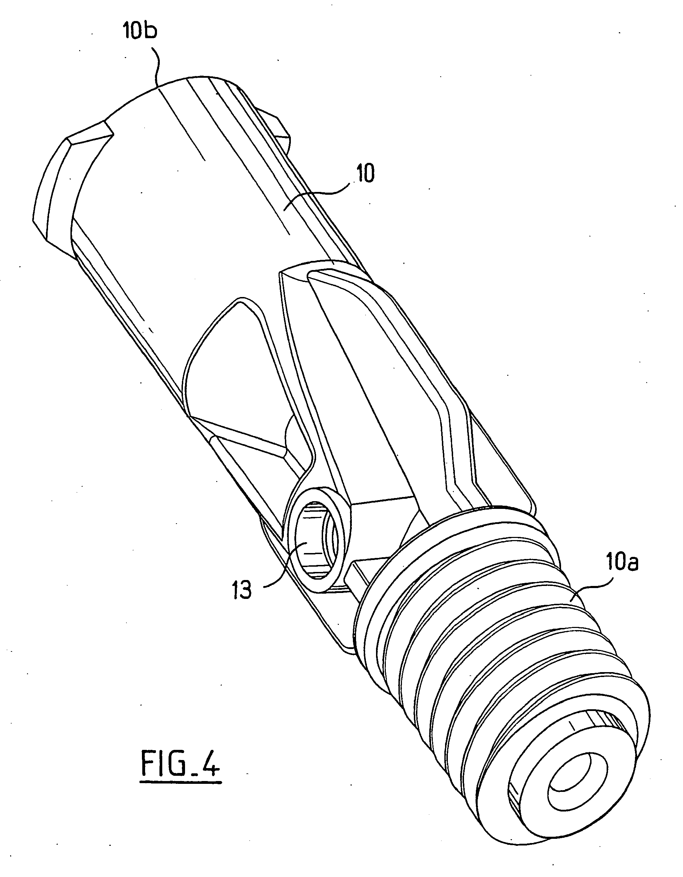

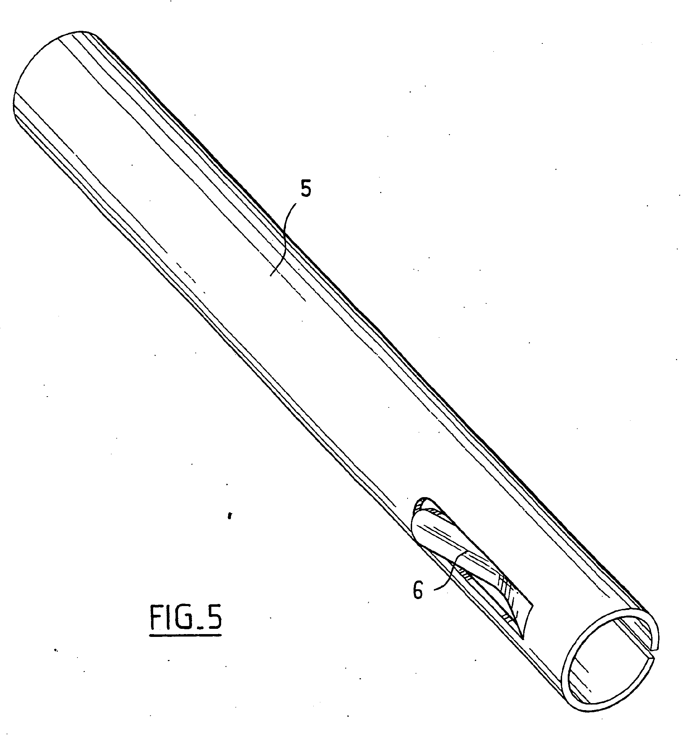

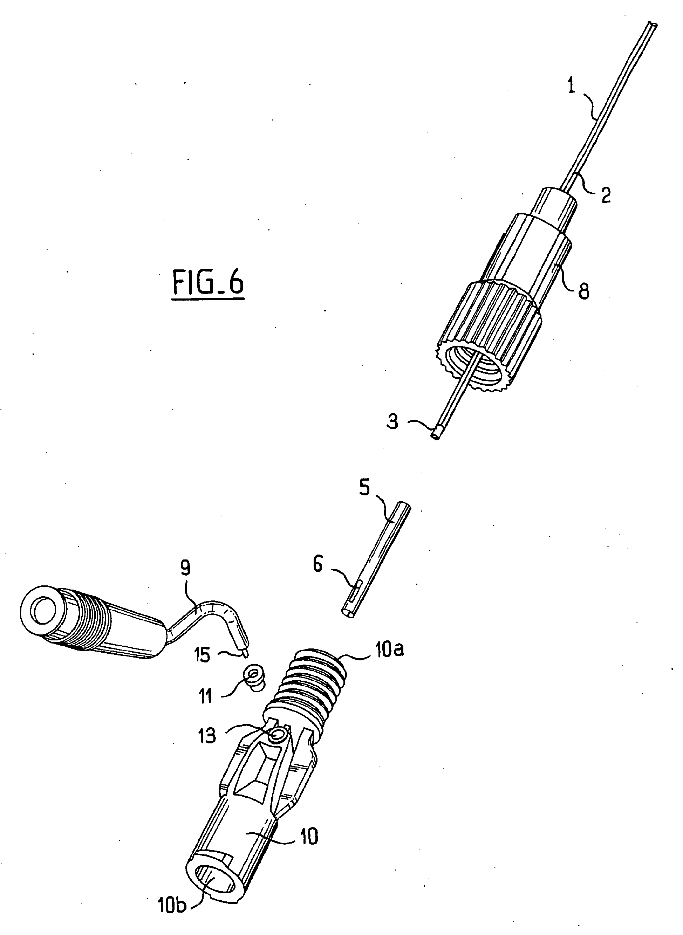

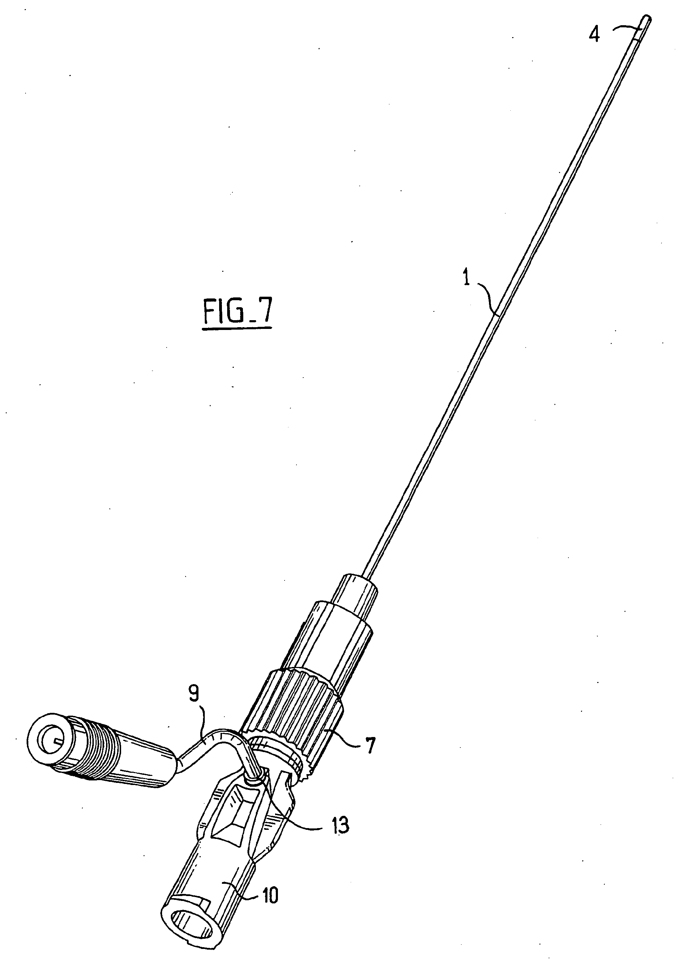

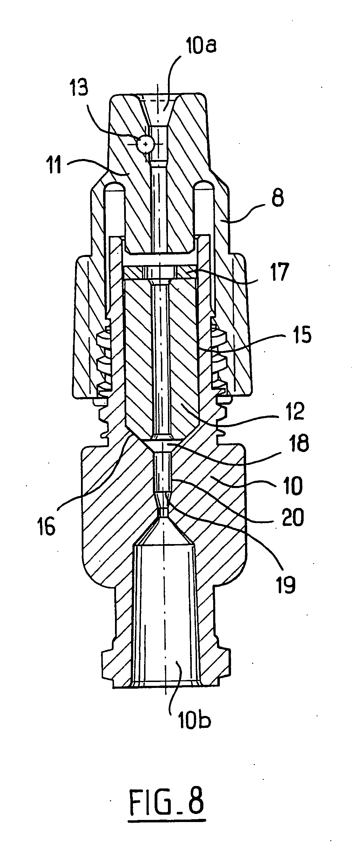

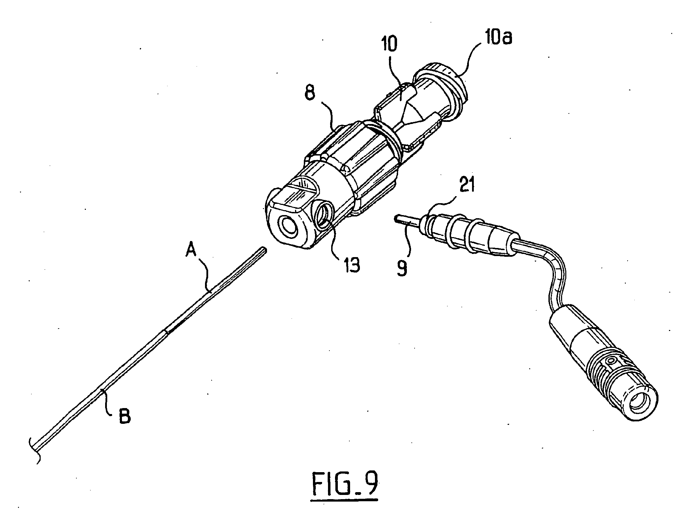

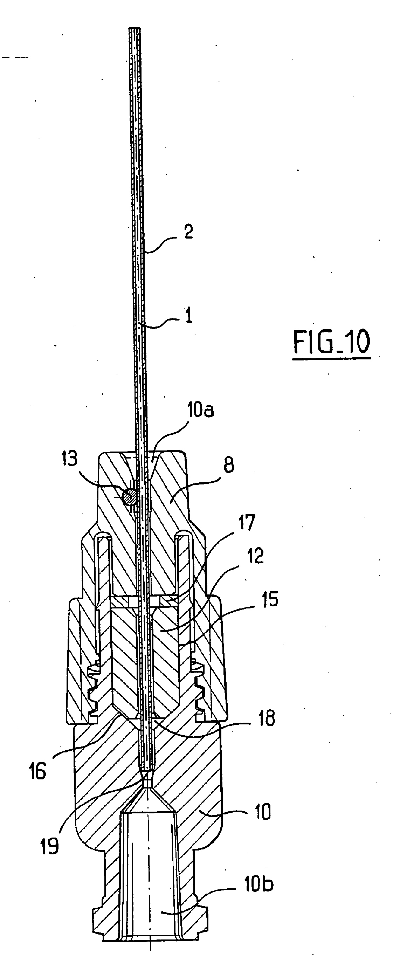

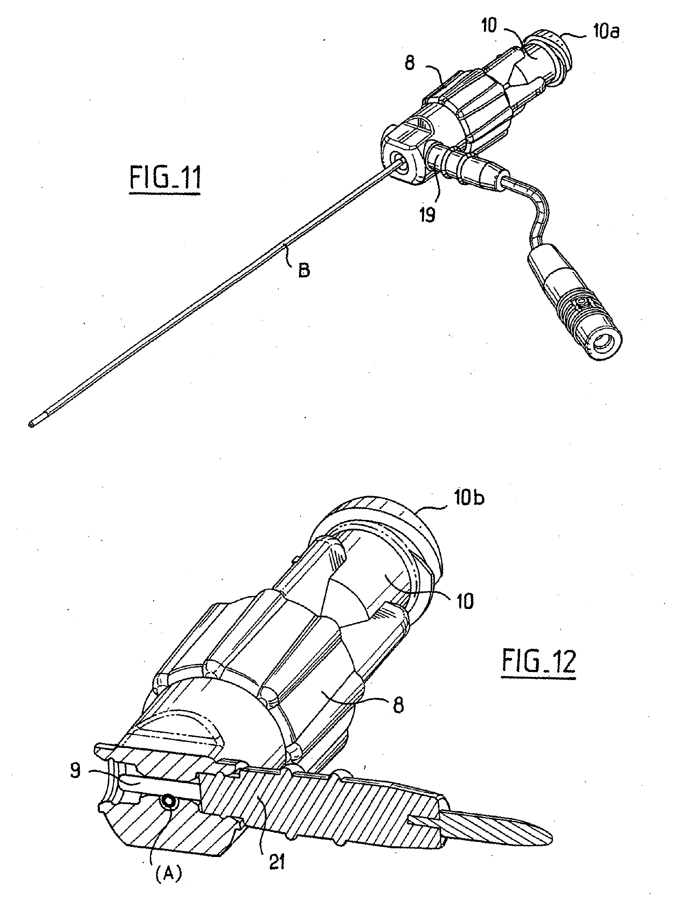

L'embase comprend un corps tubulaire (10) sur une extrémité (10a) duquel peut être vissé un écrou (11) de façon à assurer la compression d'un manchon élastomère (12), logé dans l'écrou (réalisation des figures 1 à 7) ou dans le corps tubulaire (réalisation des figures 8 à 12) et traversé par le tube cathéter lorsque celui-ci est introduit dans l'embase ; l'autre extrémité (10b) du corps tubulaire comporte un passage d'entrée conique (conicité luer) pour le raccordement de l'embout d'un dispositif de fourniture de solution.

L'écrou et le manchon coopèrent pour assurer par compression latérale du cathéter le maintien du cathéter dans l'embase.

Pour assurer la connexion électrique, l'embase présente un trou latéral (13) qui est formé dans le corps tubulaire (réalisation des figures 1 à 7) ou dans l'écrou (réalisation des figures 8 à 12) pour le passage d'un fil (14) ou d'une fiche (15) d'un conducteur électrique (9) jusqu'au contact du tube cathéter.

Pour assurer le passage du courant selon la réalisation des figures 1 à 7, on enfile sur l'extrémité proximale du tube cathéter un tube métallique (5) fendu et élastique, avec une (ou plusieurs) languettes à ressort (6) découpées dans le tube et formées en S, de manière à être dépassantes à l'intérieur et à l'extérieur du tube. De cette manière, ce tube peut être en vis-à-vis du trou latéral (13) de l'embase. Le tube cathéter est poussé dans l'embase jusqu'en butée dans l'embase. La ou les languettes du tube métallique sont en appui sur l'anneau proximal de la couche conductrice pour assurer le contact électrique. Le tube cathéter est bloqué dans l'embase par le manchon de compression (7) serré par l'écrou.

Le fil électrique dénudé (9) et recouvert d'une bague métallique (11) est poussé dans le trou latéral (13) et bloqué. Il comprime la languette extérieurement et assure le passage du courant du fil à l'anneau proximal.

La bague peut être poussée en force, ou emmanchée et maintenue par un système type vis-écrou, le trou latéral de l'embase est alors fileté extérieurement et la bague est équipée d'un écrou vissable.

Dans ce dernier cas, le fil est démontable.

Dans une variante de réalisation (figures 8 à 12) la transmission électrique entre le cathéter et le fil d'alimentation électrique est obtenue par un contact latéral direct entre le revêtement conducteur du cathéter et le fil.

Dans un exemple selon cette variante, l'extrémité proximale du tube cathéter, qui comporte la couche conductrice extérieure (A) mais qui est dépourvue de la couche isolante, est enfilée librement et directement dans l'embase (10), jusqu'à une butée (19) et est bloquée en position, par un manchon de compression (12) qui est comprimé par vissage d'un écrou (8) sur l'embase, comme précédemment .

L'embase comporte une chambre (15) de réception du manchon terminée à son extrémité proximale par une paroi conique (16).

Une bague anti-friction (17) peut être mise en place dans l'embase s'il y a trop de friction entre l'écrou et le manchon lors du vissage en compression, pour éviter que le manchon se torsade.

De fait, l'embase, le manchon et l'écrou sont pré-assemblé (sans serrage) avant introduction du cathéter dans l'embase.

Si l'embase est transparente, on peut observer la butée du cathéter dans l'embase.

Toute la longueur de cathéter introduite dans l'embase est conductrice. Au-delà de l'embase, le cathéter est recouvert du revêtement électriquement isolant (B).

On visse l'écrou pour comprimer le manchon et bloquer le cathéter. L'étanchéité est obtenue en partie proximale de l'embase. Le liquide poussé dans le luer femelle de l'embase arrive jusque dans le cathéter. Si du liquide s'introduit entre le cathéter et la paroi latérale de la lumière (20), il est recueilli dans une très petite chambre (18) dont l'étanchéité est assurée par le manchon (12) comprimé contre le cône (16) de la chambre (15) et contre le cathéter.

Après blocage de l'écrou, on introduit la fiche conductrice (19) de la ligne électrique qui va amener le courant électrique dans un trou latéral (13) de l'écrou. La fiche vient au contact de la surface extérieure conductrice du cathéter. Un clippage de l'embase (21) de cette fiche dans le trou de l'écrou est possible.

Pour réaliser la ligne conductrice, on utilise de préférence une encre conductrice à base d'argent. L'argent est un matériau idéal pour sa conductivité (sa résistivité est très basse) et sa souplesse (il suit sans problème les courbures du tube). A titre indicatif, en épaisseur de 15 à 20µm sur toute la surface du tube cathéter (φ 0,40 x 0,83 mm), on obtient une résistance d'environ 8 ohms. Or compte tenu du passage de quelques mA en neurostimulation, il vaut mieux avoir une résistance la plus faible possible.

Cette encre peut être un mélange de solvant et de poudre d'argent très fine, pour une très bonne conductivité électrique.

L'application peut être réalisée par sérigraphie, tampographie, voire au jet d'encre. Ces méthodes d'application ne sont pas limitatives.

L'invention n'est pas limitée aux réalisations qui ont été décrites.

## Revendications

1. Cathéter de neurostimulation constitué par un tube souple (1) en matériau non conducteur de l'électricité dont l'extrémité distale est arrondie et qui comprend un conducteur électrique (2) qui s'étend dans la longueur du tube pour conduire un courant électrique de stimulation d'une extrémité à l'autre du tube, ce conducteur est constitué par au moins une couche (2) d'un matériau (A) conducteur de l'électricité appliquée sur la face externe de la paroi latérale du tube (1) et isolée par un revêtement (B) non conducteur appliqué sur la couche, **caractérisé en ce que** ladite couche conductrice (2) formant à l'extrémité distale du tube un anneau distal (4) qui épouse la forme de l'arrondi de l'extrémité distale du tube et qui n'est pas recouvert par le revêtement isolant (B) de la couche de façon à constituer un contact de neurostimulation, et ladite couche conductrice (2) formant à l'extrémité proximale du tube un anneau proximal (3) qui n'est pas recouvert par le revêtement isolant (B)pour réaliser une connexion électrique.

2. Cathéter selon la revendication 1 dans lequel ladite couche conductrice (2) constitue une ligne conductrice des largeur et d'épaisseur calibrées.

3. Cathéter selon la revendication 1 ou 2 dont ladite couche est constituée par une encre contenant de l'argent.

4. Cathéter selon la revendication 1 ou 2 dont ladite couche est constituée par un mélange de solvant et de poudre d'argent.

5. Cathéter selon l'une des revendications 1 à 4 dont ladite couche a été appliquée par sérigraphie, par tampographie ou par jet d'encre.

6. Cathéter selon la revendication 1 et qui comprend un tube conducteur fendu (5) enfilé sur l'extrémité proximale (1a) du cathéter (1) pour réaliser une connexion électrique avec l'anneau proximal.

7. Cathéter selon la revendication 6 dans lequel la paroi du tube conducteur (5) est découpée longitudinalement pour former au moins une languette élastique (6) destinée à assurer un contact électrique entre le tube et un fil électrique (9) maintenu au contact de la languette.

8. Cathéter selon l'une des revendications 1 à 7 et qui comporte une embase proximale (10) équipée de moyens (8, 12), pour maintenir le cathéter dans l'embase et de moyens (13) pour assurer une connexion électrique entre un conducteur d'alimentation électrique et le conducteur du cathéter, et étant apte au raccordement du cathéter avec un dispositif d'alimentation d'une solution.

9. Cathéter selon la revendication 8 dont l'embase comporte un corps tubulaire (10) sur une extrémité duquel peut être vissé un écrou (11) de façon à assurer la compression d'un manchon élastomère (12) logé dans l'écrou ou dans le corps tubulaire et traversé par le tube cathéter lorsque celui-ci est introduit dans l'embase par ladite extrémité (10a) de l'embase, l'autre extrémité (10b) du corps tubulaire comportant un passage d'entrée conique pour le raccordement de l'embout d'un dispositif de fourniture de solution.

10. Cathéter selon la revendication 9 dont ledit manchon (12) est logé dans l'écrou (11).

11. Cathéter selon la revendication 9 dont ledit manchon est logé dans le corps (10) de l'embase.

12. Cathéter selon l'une des revendications 8 à 11 dont lesdits moyens pour assurer une connexion électrique entre le revêtement conducteur du tube et un conducteur d'alimentation comprennent un trou latéral (13) formé dans l'embase pour l'introduction du conducteur d' alimentation (9) dans l'embase.

13. Cathéter selon la revendication 12 dont le trou latéral (13) est formé dans le corps (10) de l'embase.

14. Cathéter selon la revendication 12 dont le trou latéral (13) est formé dans l'écrou (11) de l'embase.

## Claims

1. A neurostimulation catheter formed by a flexible tube (1) in a non-electricity conducting material, the distal end of which is rounded and which comprises an electric conductor (2) which extends along the length of the tube in order to conduct an electric stimulation current from one end to the other end of the tube, this conductor is formed by at least one layer (2) of an electricity-conducting material (A) applied on the external face of the side wall of the tube (1) and insulated by a non-conducting coating (B) applied on the layer, **characterized in that** said conducting layer (2) forming at the distal end of the tube, a distal ring (4) which fits the shape of the roundness of the distal end of the tube and which is not covered by the insulating coating (B) of the layer so as to form a neurostimulation contact, and said conducting layer (2) forming at the proximal end of the tube a proximal ring (3) which is not covered by the insulating coating (B), for making an electric connection.

2. The catheter according to claim 1 wherein said conducting layer (2) forms a conducting line with calibrated width and thickness.

3. The catheter according to claim 1 or 2, whereof said layer is formed by an ink containing silver.

4. The catheter according to claim 1 or 2, whereof said layer is formed by a mixture of solvent and silver powder.

5. The catheter according to any of claims 1 to 4, whereof said layer was applied by screen-printing, by pad-printing or by ink jet.

6. The catheter according to claim 1 and which comprises a slit conducting tube (5) slipped onto the proximal end (1a) of the catheter (1) in order to make an electric connection with the proximal ring.

7. The catheter according to claim 6, wherein the wall of the conducting tube (5) is cut out longitudinally in order to form at least one elastic tab (6) intended to ensure electric contact between the tube and an electric wire (9) held in contact with the tab.

8. The catheter according to any of claims 1 to 7 and which includes a proximal base (10) equipped with means (8, 12), for holding the catheter in the base and means (13) for ensuring an electric connection between an electric power supply conductor and the conductor of the catheter, and being capable of connecting the catheter with a device for feeding a solution.

9. The catheter according to claim 8, the base of which includes a tubular body (10) on an end of which a nut (11) may be screwed so as to ensure the compression of an elastomer sleeve (12) housed in the nut or in the tubular body and crossed by the catheter tube when the latter is introduced into the base through said end (10a) of the base, the other end (10b) of the tubular body including a conical entry passage for connecting the endpiece of a solution-providing device.

10. The catheter according to claim 9, whereof said sleeve (12) is housed in the nut (11).

11. The catheter according to claim 9, whereof said sleeve is housed in the body (10) of the base.

12. The catheter according to any of claims 8 to 11, whereof said means for ensuring an electric connection between the conducting coating of the tube and a power supply conductor comprise a side hole (13) formed in the base for introducing the power supply conductor (9) into the base.

13. The catheter according to claim 12, whereof the side hole (13) is formed in the body (10) of the base.

14. The catheter according to claim 12, whereof the side hole (13) is formed in the nut (11) of the base.

## Patentansprüche

1. Katheter zur Neurostimulation, der aus einem biegsamen Schlauch (1) aus einem elektrisch nicht leitfähigen Werkstoff besteht, wobei dessen distales Ende abgerundet ist und er einen elektrischen Leiter (2) umfasst, welcher sich entlang des Schlauches erstreckt, um einen elektrischen Stimulationsstrom von einem Ende des Schlauches zum anderen zu leiten, wobei der Leiter aus mindestens einer Schicht (2) eines elektrisch leitfähigen Werkstoffs (A) besteht, welche auf die Außenfläche der Seitenwand des Schlauches (1) aufgebracht ist und durch eine nicht leitfähige Beschichtung (B), die auf die Schicht aufgebracht ist, isoliert ist, **dadurch gekennzeichnet, dass** die leitfähige Schicht (2) am distalen Ende des Schlauches einen distalen Ring (4) bildet, welcher der Form der Abrundung des distalen Endes des Schlauches angepasst ist und welcher nicht von der isolierende Beschichtung (B) der Schicht bedeckt ist, sodass ein Kontakt zur Neurostimulation hergestellt wird, und dass die leitfähige Schicht (2) am proximalen Ende des Schlauches einen proximalen Ring (3) bildet, welcher nicht von der isolierenden Beschichtung (B) bedeckt ist, um eine elektrische Verbindung herzustellen.

2. Katheter nach Anspruch 1, wobei die leitfähige Schicht (2) eine leitfähige Linie mit festgelegter Breite und Dicke bildet.

3. Katheter nach Anspruch 1 oder 2, wobei dessen Schicht aus einer Druckfarbe besteht, die Silber enthält.

4. Katheter nach Anspruch 1 oder 2, wobei dessen Schicht aus einer Mischung aus Lösungsmittel und Silberpulver besteht.

5. Katheter nach einem der Ansprüche 1 bis 4, wobei dessen Schicht durch Siebdruck, durch Tampondruck oder durch Tintenstrahldruck aufgebracht wurde.

6. Katheter nach Anspruch 1, der weiterhin einen geschlitzten leitfähigen Schlauch (5) umfasst, welcher auf das proximale Ende (1a) des Katheters (1) aufgeschoben ist, um eine elektrische Verbindung mit dem proximalen Ring herzustellen.

7. Katheter nach Anspruch 6, wobei die Wand des leitfähigen Schlauches (5) in Längsrichtung einschnitten ist, um mindestens eine elastische Zunge (6) zu bilden, die dazu bestimmt ist, einen elektrischen Kontakt zwischen dem Schlauch und der elektrischen Draht (9), der ständig mit der Zunge in Kontakt ist, sicherzustellen.

8. Katheter nach einem der Ansprüche 1 bis 7, der weiterhin einen proximalen Sockel (10) umfasst, welcher mit Mitteln (8, 12) zum Festhalten des Katheters im Sockel und mit Mitteln (13) zum Sicherstellen einer elektrischen Verbindung zwischen einem Stromversorgungsleiter und dem Leiter des Katheters versehen ist und welcher dazu eignet ist, den Katheter an eine Vorrichtung zur Versorgung mit einer Lösung anzuschließen.

9. Katheter nach Anspruch 8, wobei dessen Sockel einen röhrenförmigen Körper (10) umfasst, wobei eine Mutter (11) derart auf ein Ende desselben geschraubt werden kann, dass die Kompression einer Elastomerhülse (12), die sich in der Mutter oder im röhrenförmigen Körper befindet und die vom Katheterschlauch durchquert wird, wenn dieser über das Ende (10a) des Sockels in den Sockel eingeführt wird, sichergestellt wird, wobei das andere Ende (10b) des röhrenförmigen Körpers eine konische Eintrittsöffnung aufweist, um das Ansatzstück einer Vorrichtung zur Bereitstellung einer Lösung anzuschließen.

10. Katheter nach Anspruch 9, wobei dessen Hülse (12) in der Mutter (11) angeordnet ist.

11. Katheter nach Anspruch 9, wobei dessen Hülse im Körper (10) des Sockels angeordnet ist.

12. Katheter nach einem der Ansprüche 8 bis 11, wobei dessen Mittel zum Sicherstellen einer elektrischen Verbindung zwischen der leitfähigen Beschichtung des Schlauches und einem Versorgungsleiter ein seitliches Loch (13) umfassen, das im Sockel gebildet ist, um den Versorgungsleiter (9) in den Sockel einzuführen.

13. Katheter nach Anspruch 12, wobei dessen seitliches Loch (13) im Körper (10) des Sockels gebildet ist.

14. Katheter nach Anspruch 12, wobei dessen seitliches Loch (13) in der Mutter (11) des Sockels gebildet ist.
